# EUROPEAN PATENT SPECIFICATION

(11) **EP 0 267 851 B1**
(45) Date of publication and mention of the grant of the patent: **19.01.1994**
(21) Application number: 87402542.2
(22) Date of filing: 10.11.1987
(51) Int. Cl.: C12N 15/70

(54) **Hybrid promoter, expression controlling DNA sequence and expression vector**
Hybrid-Promotor, expressionskontrollierende DNS-Sequenz und Expressions-Vektor
Promoteur hybride, séquence d'ADN contrôlant l'expression et vecteur d'expression

(30) Priority: 11.11.1986 JP 268362/86
(43) Date of publication of application: 18.05.1988
(73) Proprietor: MITSUBISHI KASEI CORPORATION, Tokyo 100 (JP)
(72) Inventor: Shibui, Tatsurou, Machida-shi Tokyo (JP); Kamizono, Michiru, Machida-shi Tokyo (JP); Teranishi, Yutaka, Sagamihara-shi Kanagawa-ken (JP)
(74) Representative: Gutmann, Ernest

(56) References cited:
- EP-A- 0 152 613
- EP-A- 0 186 069
- DD-A- 237 675
- US-A- 4 495 280
- US-A- 4 689 406
- CHEMICAL ABSTRACTS, vol. 100, no. 23, 4th June 1984, abstract no. 186532m, Columbus, Ohio, US; V.N. DOBRYNIN et al.: "Synthetic promoter P25 of the bacteriophage T5 and its functional hybrids with the model promoter of Escherichia coli"; & Dokl. Akad. Nauk SSSR, 1984, vol. 274, no. 1, pp. 213-216
- E.L.Winnacker ,1985, "Gene und Klone", VCH-Weinhem, FRG, page 196

## Description

The present invention relates to a hybrid promoter which allows for efficient expression, to an expression-controlling DNA sequence comprising said hybrid promoter and to an expression vector comprising said expression-controlling DNA sequence.

Expression of genes takes place in two steps, i.e., transcription and translation. In transcription step an RNA polymerase specifically recognizes "-35 region" located approximately 35 base pairs upstream of the start of mRNA synthesis within a region, called promoter, which spans about 50 base pairs and is located upstream of a structural gene, and then binds to "-10 region" located about 10 base pairs upstream of the start of mRNA synthesis so as to start mRNA synthesis which is stopped at a site called terminator. The subsequent translation step is a protein synthesizing process according to triplet codons, which is initiated when a ribosome and the like recognize and bind to both a specific sequence called SD (Shine-Dalgarno) sequence and an initiation codon such as AUG on mRNA and is stopped at a termination codon.

Except for phenomena peculiar to higher organisms, such as splicing, the major part of the species specificities in gene expression exists in the specific sequences at the starting points of both transcription and translation and in the recognition processes thereof, whereas the subsequent elongation reactions generally have no specificity. Accordingly, a desired or target protein can be synthesized by incorporating a region including the promoter and the initiation codon which are responsible for the initiation of gene expression, into a plasmid cloning vector and then ligating the structural gene encoding the desired protein downstream to the region.

On expressing the desired protein using such an expression vector as described above, expression efficiency is very critical, especially from an industrial viewpoint.

There have hitherto been proposed various expression vectors using promoters such as a tac promoter, P_{L}P_{R} promoters and an expression-controlling DNA sequence consisting of a T5 phage promoter (-35 region and -10 region) and a lac operator (Japanese Patent Application Laying Open No. 61-181386) : see also Herman A. de Boer et al, Proc. Natl. Acad. Sci. USA, vol 80, pp. 21-25, January 1983.

Reference can also be made to patent DD-A1-237675 which disclosed a promoter consisting of a combined unit comprising the 5'-terminal portion of the "-35" region of the A3 promoter of phage T7 and of the "-10" region of the E. coli β-lactamase gene of pBR322.

The "-35" region of the A3 promotor of phage T7 contains of the same sequence as the bacterial trp promoter, e.g. "5' GTTGACAA 3'".

The present invention provides an expression vector which enables efficient expression in a well-controlled manner. The present invention also provides a hybrid promoter which has, as an RNA polymerase recognition region, a base sequence equivalent to the base sequence of a promoter derived from a phage and, as an RNA polymerase binding region, a base sequence equivalent to the base sequence of a promoter derived from E. coli. The present invention further provides an expression-controlling DNA sequence comprising said hybrid promoter and an operator region, a base sequence of which is equivalent to the base sequence of an operator region derived from E. coli.

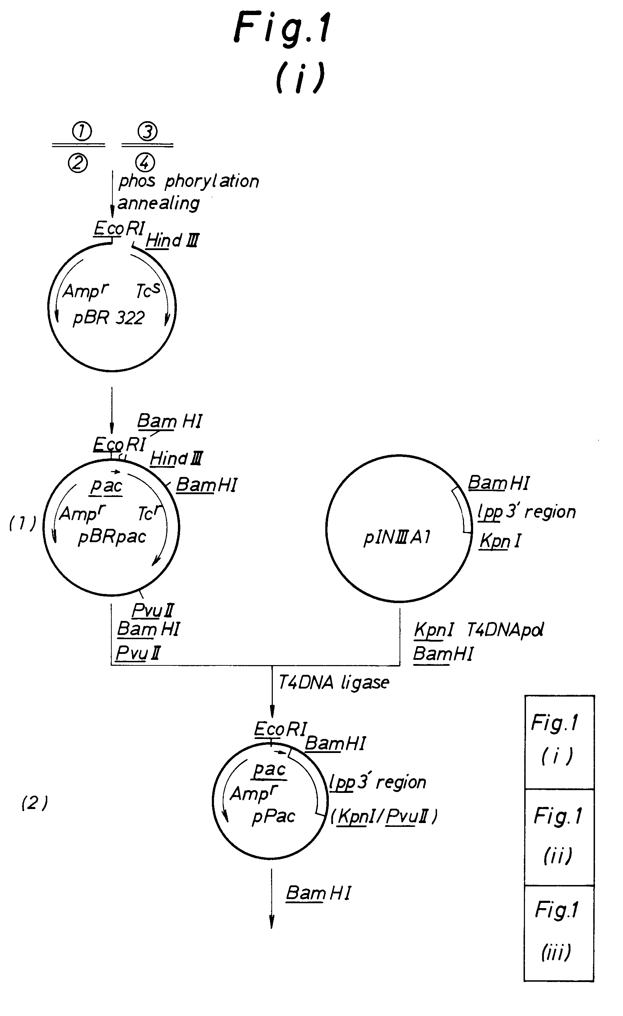

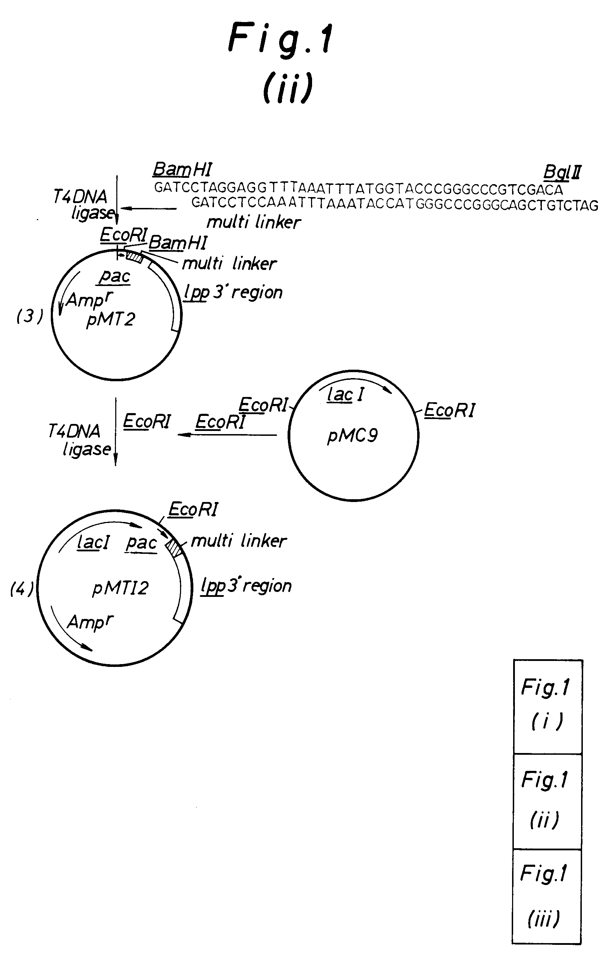

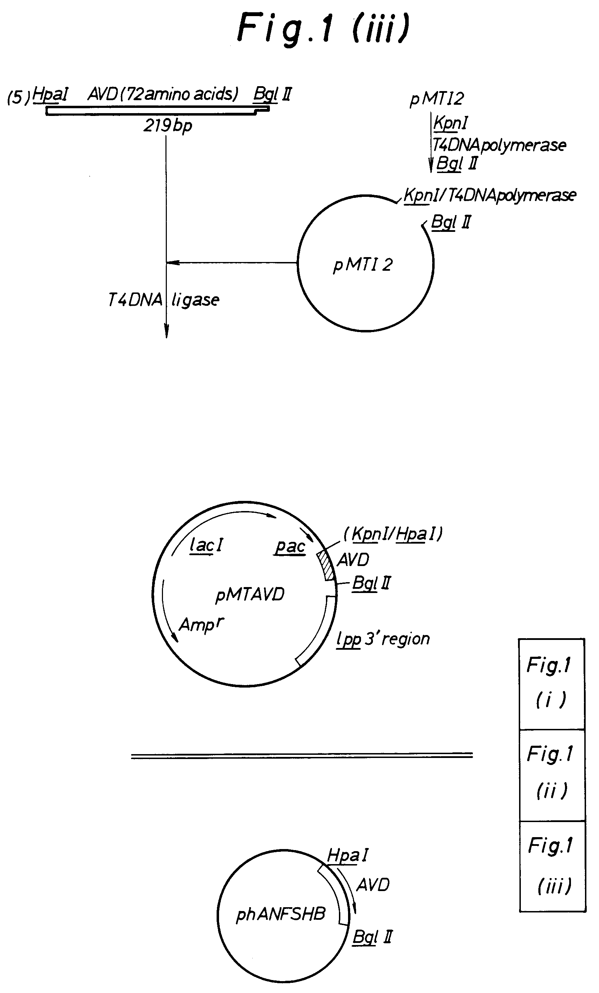

Figure 1 shows schematically the method of construction of an expression vector according to the present invention.

The hybrid promoter of the present invention comprised an RNA polymerase recognition region having a nucleotide sequence equivalent to that comprising the -35 region of a phage promoter comprising at least the following nucleotide sequence : 
5′ T T G C T 3′
3′ A A C G A 5′
and an RNA polymerase binding region having a nucleotide sequence equivalent to that of the -10 region of E. coli promoter comprising at least the following necleotide sequence : 
-10
5' T A T A A T G T G T G G 3'
3' A T A T T A C A C A C C 5'
Among the phage promoters which comprise the abovesaid base sequence in their -35 regions, there may be mentioned the promoters of P25, P26, P28 or P207 of T5 phage.

The base sequence corresponding to the -10 region in the promoters of lac UV5, rrnDₗ or rrnEₗ of E. coli may be exemplified.

According to the present invention, the -35 region is located upstream of the -10 region, wherein both regions may preferably be bound to each other through any optional base sequence of about 10 - 20 base pairs although it is also possible to have them ligated directly with each other.

The expression-controlling DNA sequence of the present invention comprises the above-mentioned hybrid promoter and the base sequence equivalent to that of the operator region derived from E. coli.

The base sequence of corresponding to the regions of lac UV5, trp or rec A of E. coli may be illustrated. 
5' AATTGTGAGCGGATAACAATT 3' ..... (d)
3' TTAACACTCGCCTATTGTTAA 5'
For example, any DNA fragment comprising the base pairs shown by (d) below may be used as an operator region.

The operator region of the present invention is normally incorporated in the neighborhood of the -35 region and the -10 region, though its location is by no means restricted thereto. It is preferably located downstream of the -10 region via any optional base sequence of 0 - 10 base pairs and is more preferably located immediately downstream of the -10 region.

The above-mentioned -35 region, and -10 region and operator region may be respectively prepared from the above phage, and E. coli, or alternatively may be prepared by chemical synthesis.

The expression-controlling DNA sequence is normally used in combination with, for example, SD sequence which is a ribosome binding region, an initiation codon, a termination codon and a repressor region. It is especially preferable to combine immediately downstream from the initiation codon a (multi-) cloning site in 6 - 50 nucleotide length consisting of a DNA sequence providing at least one or, preferably, a plurality of restriction enzyme sites which may be advantageously used in insertion of the genes encoding various target proteins. Interferons of every kind, IL-2, apolipoproteins A and E, TNF, cardiodilatin, cardionatrin and the like may be illustrated as unlimited examples of the target protein.

The above-mentioned (multi-) cloning site is illustrated, for example, by a DNA fragment having a plurality of restriction sites as shown below. 
Kpn Sma Apa Sal Bgl II Ava
5' ATGGTACCCGGGCCCGTCGACAGATCTGGG—
3' TACCATGGGCCCGGGCAGCTGTCTAGACCC—
fMet

GCA 3'
CGTCTAG 5'
The expression vector of the present invention comprises the gene encoding the target protein and a terminator inserted downstream of the expression-controlling DNA sequence described above. Among the terminators there may be mentioned the 3ʹ region of lipoprotein (lpp) gene, the terminators of the trp operon and rRNA gene, and the t₀ of λ phage.

The hybrid promoter and the expression-controlling DNA sequence of the present invention make it possible to efficiently synthesize the target protein in a well-controlled fashion.

The present invention will be described more in detail, but without limitation, in the following example.

### Example:

### (1) Construction of an expression-controlling DNA sequence

A DNA fragment consisting of the same sequences as those of the -10 region, the operator region and the transcription initiation point of the lac UV5 promoter, and a DNA fragment consisting of the same sequence as that of the -35 region of the T5 pharge P25 promoter ((1) - (4) shown below) were synthesized using a DNA synthesizer Model 308 A (Nikkaki Co., Japan) 
①5′AATTCAAAAATTTATTTGCTTTCAGGAAAATTTTT
CT3′

②5′ATACAGAAAAATTTTCCTGAAAGCAAATAAATTTT
TG3′

③5′GTATAATGTGTGGAATTGTGAGCGGATAACAATTT
CGGATCCA3′

④5′AGCTTGGATCCGAAATTGTTATCCGCTCACAATTC
CACACATT3′

EcoR I

−35
①5′AATTCAAAAATTTATTTGCTTTCAGGAAAATTTTTCT3′
3′GTTTTTAAATAAACGAAAGTCCTTTTAAAAAGACATA5′②

−10 Operator BamHI HindIII
③5′GTATAATGTGTGGAATTGTGAGCGGATAACAATTTCGGATCCA3′
3′TTACACACCTTAACACTCGCCTATTGTTAAAGCCTAGGTTCGA5′④
(1) 37 bases: a DNA fragment corresponding to the DNA fragment comprising the -35 region of T5 phage P25 promoter.
(2) 37 bases: a DNA fragment complementary to (1).
(3) 43 bases: a DNA fragment corresponding to the DNA fragment comprising -10 region and the operator region of lac UV5.
(4) 43 bases: a DNA fragment complementary to (3).

Each synthesized DNA fragment in an amount of 100 µg was incubated in 2 ml of 28% ammonium aqueous solution at 55°C for 6 hours for deprotection and purified by 17% acrylamide - 7M urea gel electrophoresis so as to obtain 10 µg of each purified DNA fragment. Subsequently, 1 µg of each DNA fragment was incubated with one unit of T4 polynucletidekinase in 10 µl of a buffer solution containing 10mM Tris-HCl, pH 7.6 and 10mM MgCl₂ at 37°C for one hour in order to phosphorylate the 5ʹ-terminus thereof. After addition of NaCl to the buffer solution to a final concentration of 50mM, the DNA fragment (1) was mixed with the fragment (2), and the fragment (3) was mixed with the fragment (4), respectively. Then, each mixture was heated at 100°C for 3 minutes and gradually cooled for annealing to result in 2 µg each of double-stranded DNA fragments of 37 bp and 43 bp, respectively.

The double-stranded DNA fragments of 0.5µg each were inserted together into 1 µg of plasmid pBR 322 treated with Eco RI and Hind III using 1 unit of T4 DNA ligase in 10 µl of a buffer solution containing 10mM Tris-HCl, pH 7.5, 6mM MgCl₂, 10mM ATP and 1mM DTT at 4°C for 16 hours.

To this reaction mixture were added CaCl₂ to a final concentration of 100mM. The resultant mixture was mixed with competent cells of E. coli HB101 and incubated at 0°C for 10 minutes and then at 37°C for 5 minutes to carry out transformation of E. coli HB101. The transformant thus obtained acquired tetracycline resistance and could be easily selected by adding tetracycline in a final concentration of 20 µg/ml to a medium.

Plasmid pBRpac with pac promoter activity was thus obtained. The base sequence of the pac promoter was determined by a conventional method, i.e., the dideoxy method, which is referred to in detail in Anal. Biochem Vol. 152,232. The competent cells may be obtained by collecting, in a conventional manner, the cells in their logarithmic phase, suspending them in a 1/3 volume of 0.1M CaCl₂ at 0°C, allowing the suspension to stand for 30 minutes, and recovering the cells in the suspension and again suspending them in a 1/100 volume of 0.1M CaCl₂.

### (2) Incorporation of a terminator into the expression-controlling DNA sequence

One µg of the plasmid pBRpac (4.4 kbp) obtained above was digested with 1 unit each of BamHI and PvuII in 10 µl of a buffer solution containing 10mM Tris-HCl, pH 7.5, 100mM NaCl and 6mM MgCl₂ by incubating the mixture at 37°C for 2 hours. The reactant was precipitated with ethanol and evaporated to dryness for preservation.

On the other hand, 5 µg of plasmid pINIIIA1 (The EMBOJ, Vol. 3, 10, 2437, 1984) was digested with 5 units Kpn I in 50 µl of a buffer solution containing 10 mM Tris-HCl, pH 7.5 and 6nM MgCl₂ by incubating the mixture at 37°C for 2 hours. To the mixture was added 5.5 µl of a buffer solution containing 330 mM Tris-Ac, pH7.9, 660mM KAc and 100mM Mg (Ac)₂ and the protruding 3ʹ termini of the digested pINIIIA1 were removed by incubation with 10 units of T4DNA polymerase at 37°C for 15 minutes. The polymerase was inactivated by heating at 70°C for 10 minutes. To this reaction mixture was then added 6.0 µl of a buffer solution containing 100mM Tris-HCl, pH 7.5, 1M NaCl and 60mM MgCl₂ and digestion with 5 units of BamHI was carried out at 37°C for 2 hours, followed by the addition of 60 µl of an aqueous saturated phenol solution for extraction of proteins. After removal of proteins, ethanol precipitation was repeated three times to result in 0.5 µg of lpp 3ʹ region (∼500 bp) fragment comprising the terminator. This DNA fragment was inserted into said digested plasmid (pBRpac) using one unit of T4DNA ligase in 10 µl of a buffer solution containing 10mM Tris-HCl, pH7.5, 10mM MgCl₂, 10mM ATP and 1mM DTT by incubating the mixture at 4°C for 16 hours, resulting in plasmid pPac.

The plasmid pPac may be stably maintained in JM 105 or JM 109 which is lac I^{q} strain. Thus 1 µg of pPac was used to transform JM 105 strain according to the method described in Example, (1). The resulting transformants were grown in 1 litre of a minimal medium containing 20 mg/ml of ampicillin and 1 mg of pPac was purified in a conventional manner.

### (3) Incorporation of a multi-linker

A multi-linker, i.e., multi-cloning site, combined with SD sequence and the initiation Met sequence, as shown in Fig. 1, was synthesized using Model 308A (Nikkaki Co.) and purified according to the method described in Example, (1) to obtain 10 µg of DNA.

On the other hand, 1 µg of the aforementioned plasmid pPac was cleaved with 1 unit of BamHI in 10 µl of a buffer solution containing 10mM Tris-HCl, pH7.5, 100 mM NaCl and 6mM MaCl₂ at 37°C for 2 hours, precipitated with ethanol and evaporated to dryness for preservation.

This cleaved plasmid pPac and 1 µg of the above multi-linker were incubated with 1 unit T4DNA ligase in 10 µl of a buffer solution containing 10mM Tris-HCl, pH 7.5, 10mM MgCl₂, 10mM ATP and 1mM DTT at 4°C for 16 hours to obtain plasmid pMT2 with the multi-linker inserted therein.

(4) Incorporation of a lac repressor gene

10 µg of plasmid pMC9 (Proc. Natl. Acad. Sci. USA, 80, 3015 (1983)) was cleaved with 10 units of EcoRI in 100 µl of a buffer solution containing 100mM Tris-HCl, pH 7.5, 10mM NaCl and 6mM MgCl₂ by incubating the mixture at 37°C for 2 hours, precipitated with ethanol and then evaporated to dryness for preservation. As a result, 2.5 µg of 1.7 Kbp DNA fragment comprising lac I, i.e., a repressor gene was obtained.

On the other hand, 1 µg of the aforementioned plasmid pMT2 was cleaved with 1 unit EcoRI in 10 µl of a buffer solution containing 100mM Tris-HCl, pH 7.5, 10mM NaCl and 6mM MgCl₂ by incubating the mixture at 37°C for 2 hours.

The cleaved plasmid pMT2 and 1 µg of the 1.7 Kbp DNA fragment were ligated with each other by the use of 1 unit of T4DNA ligase in 20 µl of a buffer solution containing 10mM Tris-HCl pH 7.5, 10mM MgCl₂, 10mM ATP and 1mM DTT to give plasmid pMTI2.

### (5) Construction of an expression plasmid

After cleaving 1 µg of pMTI2 in 10 µl of a buffer solution containing 10mM Tris-HCl pH7.5, 6mM MgCl₂ and 1 unit Kpn I at 37°C for 2 hours, 1 µl of a buffer solution containing 330mM Tris-Ac pH 7.9, 660mM KAc and 100mM Mg (Ac)₂ was added to the reaction mixture. The protruding 3ʹ termini of the digested pMTI2 were then removed by treatment with 1 unit of T4DNA polymerase in the reaction mixture at 37°C for 15 minutes and the polymerase was inactivated by heating at 70°C for 10 minutes. To the reaction mixture was further added 1.2 µl of a buffer system containing 100mM Tris-HCl, pH7.5, 1M NaCl and 60mM MgCl₂ and digestion with 1 unit of Bgl II was carried out at 37°C for 2 hours. After treatment with phenol for removal of proteins, ethanol precipitation was repeated three times to result in precipitate, which was then evaporated to dryness for preservation.

Subsequently, 1 µg of the HpaI-BglII fragment (219 bp) of the gene of AVD (Arterial Vaso Dilatin), i.e., an atriovasodilation peptide, and the plasmid pMTI2 treated as above, were linked with each other in 10 µl of a buffer solution containing 10mM Tris-HCl, pH7.5, 10mM MgCl₂, 10mM ATP and 1mM DTT using 1 unit of T4DNA ligase to obtain plasmid pMTAVD. E. coli YA21 was transformed with 0.1 µg of this pMTAVD according to the method described in Example, (1).

The resulting transformant was capable of producing approximately 36 mg/l of AVD within the cells as a result of the expression of AVD, induced by adding IPTG (isopropyl-1-thio-β-D-galactoside).

The above mentioned AVD gene was obtained by digesting with HpaI and BglII plasmid phANFSHB which had been acquired from phANF66 by such a site-specific mutation that a HpaI site and a BglII site were introduced 5ʹ and 3ʹ to the AVD gene, respectively (Nature, 310, 699, (1985)).

## Claims

1. A hybrid promoter comprising an RNA polymerase recognition region having a nucleotide sequence equivalent to that comprising the -35 region of a phage promoter comprising at least the following nucleotide sequence : 
5′ T T G C T 3′
3′ A A C G A 5′
 and an RNA polymerase binding region having a nucleotide sequence equivalent to that of the -10 region of E. coli promoter comprising at least the following necleotide sequence :

5′ TATAATGTGTGG 3′
3′ ATATTACACACC 5′

2. A hybrid promoter as claimed in claim 1 wherein the RNA polymerase recognition region has a base sequence equivalent to that of the RNA polymerase recognition region of a T5 phage P25 promoter.

3. A hybrid promoter as claimed in claim 1 or 2 wherein the RNA polymerase binding region has a base sequence equivalent to that of the RNA polymerase binding region of a lac UV5 promoter.

4. An expression-controlling DNA sequence comprising the hybrid promoter according to any of claims 1 or 3, and an operator region having a base sequence equivalent to that of an E. coli operator region.

5. An expression-controlling DNA sequence as claimed in claim 4 wherein the operator region has a base sequence equivalent to that of a lac UV5 operator region.

6. An expression-controlling DNA sequence as claimed in claim 4 or 5 comprising a DNA sequence having an initiation codon and at least one restriction enzyme site located immediately downstream of said initiation codon.

7. An expression-controlling DNA sequence as claimed in claim 6 comprising the following base sequence:

AATTCAAAAATTTATTTGCTTTCAGGAAAATTTTTCTGT
GTTTTTAAATAAACGAAAGTCCTTTTAAAAAGACA

ATAATGTGTGGAATTGTGAGCGGATAACAATTTCGGATCCA
TATTACACACCTTAACACTCGCCTATTGTTAAAGCCTAGGTTCGA .

8. An expression vector wherein a gene encoding a target protein and a terminator are inserted downstream of the expression-controlling DNA sequence according to claim 4.

9. An expression vector as claimed in claim 8 wherein the operator region has a base sequence equivalent to that of a lac UV5 operator region.

10. An expression vector as claimed in claim 9 comprising a DNA sequence having an initiation codon and at least one restriction enzyme site located immediately downstream of said initiation codon.

## Patentansprüche

1. Hybrid-Promotor umfassend eine RNS-Polymerase-Erkennungs-Region mit einer Nucleotid-Sequenz, die jener der -35-Region eines Phagenpromotors entspricht, und die mindestens die folgende Nucleotid-Sequenz enthält: 
5'   T T G C T   3'
5'   A A C G A   5'
 sowie eine RNS-Bindungs-Region mit einer Nucleotidsequenz, die jener der -10-Region eines E.coli-Promotors entspricht, die mindestens die folgende Nucleotidsequenz enthält:

5' TATAATGTGTGG 3'
3' ATATTACACACC 5'

2. Hybrid-Promotor nach Anspruch 1, bei dem die RNS-Polymerase-Erkennungs-Region eine Basensequenz hat, die der einer RNS-Polymerase-Erkennungs-Region eines P25-Promotors von Phage T5 entspricht.

3. Hybrid-Promotor nach Anspruch 1 oder 2, bei dem die RNS-Polymerase-Bindungs-Region eine Basensequenz hat, die der RNS-Polymerase-Bindungs-Region eines lac UV5-Promotors entspricht.

4. Expressionkontrollierende DNS-Sequenz, die den Hybridpromotor nach einem der Ansprüche 1 oder 3 und eine Operatorregion mit einer Basensequenz, die jener einer E.coli-Operatorregion entspricht, umfaßt.

5. Expressionkontrollierende DNS-Sequenz nach Anspruch 4, in der die Operatorregion eine Basensequenz hat, die jener einer lac UV5-Operatorregion entspricht.

6. Expressionkontrollierende DNS-Sequenz nach Anspruch 4 oder 5, die eine DNS-Sequenz mit einem Initiationscodon und mindestens eine Restriktionsenzymstelle, die sich unmittelbar stromabwärts von diesem Initiationscodon befindet, umfaßt.

7. Expressionkontrollierende DNS-Sequenz nach Anspruch 6, die die folgende Basensequenz umfaßt:

AATTCAAAAATTTATTTGCTTTCAGGAAAATTTTTCTGT
GTTTTTAAATAAACGAAAGTCCTTTTAAAAAGACA

ATAATGTGTGGAATTGTGAGCGGATAACAATTTCGGATCCA
TATTACACACCTTAACACTCGCCTATTGTTAAAGCCTAGGTTCGA .

8. Expressionsvektor, in dem ein Gen, das ein Zielprotein codiert, und ein Terminator stromabwärts von der expressionkontrollierenden DNS-Sequenz nach Anspruch 4 eingebaut sind.

9. Expressionsvektor nach Anspruch 8, indem die Operatorregion eine Basensequenz hat, die jener einer lac UV5-Operatorregion entspricht.

10. Expressionsvektor nach Anspruch 9, der eine DNS-Sequenz mit einem Initiationcodon und mindestens eine Restriktionsenzymstelle, die sich unmittelbar stromabwärts von dem Initiationscodon befindet, umfaßt.

## Revendications

1. Promoteur hybride comprenant une région de reconnaissance d'ARN polymérase ayant une séquence de nucléotides équivalente à celle comprenant la région -35 d'un promoteur de phage comprenant au moins la séquence de nucléotides suivante : 
5' TTGCT 3'
3' AACGA 5'
 et une région de liaison d'ARN polymérase ayant une séquence de nucléotides équivalente à celle de la région -10 d'un promoteur d'E. coli comprenant au moins la séquence de nucléotides suivante :

5' TATAATGTGTGG 3'
3' ATATTACACACC 5'

2. Un promoteur hybride selon la revendication 1, dans lequel la région de reconnaissance d'ARN polymérase a une séquence de bases équivalente à celle de la région de reconnaissance d'ARN polymérase d'un promoteur P25 de phage T5.

3. Un promoteur hybride selon la revendication 1 ou 2, dans lequel la région de liaison d'ARN polymérase a une séquence de bases équivalente à celle de la région de liaison d'un promoteur UV5 lac.

4. Une séquence d'ADN régulatrice d'expression comprenant le promoteur hybride selon l'une quelconque des revendications 1 ou 3, et une région d'opérateur ayant une séquence de bases équivalente à celle d'une région d'opérateur d'E. coli.

5. Une séquence d'ADN régulatrice d'expression selon la revendication 4, dans laquelle la région d'opérateur a une séquence de bases équivalente à celle d'une région d'opérateur UV5 lac.

6. Une séquence d'ADN régulatrice d'expression selon la revendication 4 ou 5 comprenant une séquence d'ADN ayant un codon d'initiation et au moins un site d'enzyme de restriction situé immédiatement en aval dudit codon d'initiation.

7. Une séquence d'ADN régulatrice d'expression selon la revendication 6, comprenant la séquence de bases suivante :

AATTCAAAAATTTATTTGCTTTCAGGAAAATTTTTCTGT
GTTTTTAAATAAACGAAAGTCCTTTTAAAAAGACA

ATAATGTGTGGAATTGTGAGCGGATAACAATTTCGGATCCA
TATTACACACCTTAACACTCGCCTATTGTTAAAGCCTAGGTTCGA

8. Un vecteur d'expression dans lequel un gène codant pour une protéine cible et un terminateur sont insérés en aval de la séquence d'ADN régulatrice d'expression selon la revendication 4.

9. Un vecteur d'expression selon la revendication 8, dans lequel la région d'opérateur a une séquence de bases équivalente à celle d'une région d'opérateur UV5 lac.

10. Un vecteur d'expression selon la revendication 9, comprenant une séquence d'ADN ayant un codon d'initiation et au moins un site d'enzyme de restriction situé immédiatement en aval dudit codon d'initiation.
